Europäisches Patentamt

(19) European Patent Office

Office européen des brevets

(11) Numéro de publication : **0 082 228 B1**

(12) **FASCICULE DE BREVET EUROPÉEN**

(45) Date de publication du fascicule du brevet :
03.04.85

(51) Int. Cl.⁴ : **A 23 L 3/00**

(21) Numéro de dépôt : **81402062.4**

(22) Date de dépôt : **23.12.81**

(54) **Panier tubulaire pour la stérilisation des produits alimentaires en boîtes métalliques cylindriques.**

(43) Date de publication de la demande :
**29.06.83 Bulletin 83/26**

(45) Mention de la délivrance du brevet :
**03.04.85 Bulletin 85/14**

(84) Etats contractants désignés :
**AT BE CH DE FR GB IT LI LU NL SE**

(56) Documents cités :
**BE-A- 853 383**
**FR-A- 1 449 779**
**FR-A- 1 591 576**
**GB-A- 924 054**
**GB-A- 1 119 822**
**GB-A- 1 226 026**
**GB-A- 1 509 046**
**US-A- 1 569 601**
**US-A- 2 443 840**
**US-A- 3 710 926**
**JOURNAL OF FOOD SCIENCE, vol.47, no.1, janvier-février 1981, CHICAGO, Ill. (US) R.A. ROOP et al.: "Processing retort pouches in conventional sterilizers", pages 303-305**

(73) Titulaire : **Gomez, Robert**
**27 rue de la Liberté**
**F-93700 Drancy (FR)**

(72) Inventeur : **Gomez, Robert**
**27 rue de la Liberté**
**F-93700 Drancy (FR)**

(74) Mandataire : **Laget, Jean-Loup et al**
**Cabinet Pierre Loyer 18, Rue de Mogador**
**F-75009 Paris (FR)**

Jouve, 18, rue St-Denis, 75001 Paris, France

## Description

L'invention concerne un panier tubulaire pour la stérilisation des produits alimentaires en boîtes métalliques cylindriques.

Il est connu de procéder à la stérilisation des produits alimentaires en boîtes métalliques cylindriques dans des installations de type à colonne hydrostatique. Dans ces installations sont prévus des conteneurs ou paniers cylindriques percés de nombreux trous, qui circulent dans une enceinte remplie de vapeur saturante d'eau. Ces paniers sont déplacés dans l'enceinte par des jeux de chaînes d'entraînement. Ces chaînes assurent le déplacement des paniers dans l'enceinte selon un itinéraire bien défini en fonction du temps de stérilisation à respecter et des différentes températures imposées aux divers stades du processus de stérilisation.

Une installation de ce type est décrite dans le brevet français n° 1.454.040.

Dans les paniers tubulaires connus, les boîtes sont insérées longitudinalement, leur axe longitudinal étant confondu avec l'axe longitudinal du panier. Il en résulte deux inconvénients. Le premier est que les boîtes peuvent glisser à l'intérieur du panier. En particulier, dans le cas d'emboîtage sous vide, il y a très peu de liquide dans la boîte ou même pas du tout. La boîte a tendance à « flotter » au cours du traitement de stérilisation, et elle présente toujours la même zone vers le haut par exemple. Lorsque le panier doit tourner sur lui-même autour de son axe longitudinal, la boîte a tendance à glisser par rapport au panier et à garder toujours son orientation première. Il en résulte qu'il n'y a pas de brassage à l'intérieur de la boîte, ce qui peut rendre la stérilisation imparfaite, mais surtout amène à prolonger le temps de stérilisation par mesure de sécurité. La conséquence en est une augmentation de la consommation d'énergie de l'installation et donc un prix de revient plus élevé.

Le second inconvénient est que les boîtes voisines sont en contact entre elles par leurs faces transversales, en regard, qui sont circulaires. Au droit de ces faces transversales, il n'y a pratiquement pas d'échanges thermiques avec le milieu contenu dans l'enceinte de stérilisation.

La conséquence immédiate est que pour stériliser correctement le produit contenu dans les boîtes, il est nécessaire de prolonger le temps de séjour des boîtes dans l'enceinte, donc d'augmenter la consommation d'énergie de l'installation, c'est-à-dire, le prix de revient du traitement de stérilisation.

Il est également connu, selon le document FR-A-1 591 576, de placer des boîtes dans des paniers en fils métalliques, eux-mêmes supportés par des cornières.

Par ailleurs, selon le brevet GB 1 119 822, il est connu d'utiliser des conteneurs percés, mais fermés de tous les côtés et qui maintiennent les boîtes au moyen d'un ergot.

Afin d'éviter les inconvénients des dispositifs connus, l'invention a pour premier but de proposer un panier tubulaire assurant une rotation des boîtes liée à la rotation du panier et un échange thermique avec le milieu de stérilisation sur pratiquement toute la surface des boîtes.

Un autre but de l'invention est de permettre, grâce à l'utilisation de ce panier, une réduction du temps de stérilisation des boîtes sans diminution de la qualité de cette stérilisation, et donc un abaissement du prix de revient du traitement de stérilisation.

L'invention a pour objet un panier tubulaire pour la stérilisation des produits alimentaires en boîtes métalliques cylindriques, du type dans lequel les boîtes sont disposées jointives sur l'axe longitudinal du panier, caractérisé en ce qu'il comporte : deux extrémités cylindriques, et entre elles, une armature parallélépipédique constituée de cornières longitudinales susceptibles de recevoir les boîtes cylindriques disposées avec leur axe longitudinal perpendiculaire à l'axe longitudinal du panier, de façon à maintenir les boîtes dans une position relative fixe par rapport au panier.

D'autres caractéristiques de l'invention ressortiront de la description qui suit faite avec référence au dessin annexé sur lequel on peut voir :

Figure 1 une vue perspective d'un mode de réalisation d'un panier selon l'invention ;

Figure 2 une vue de bout du panier de la figure 1 montrant la position d'une boîte dans le panier ;

Figure 3 une vue partielle de dessus montrant la disposition relative des boîtes dans le panier.

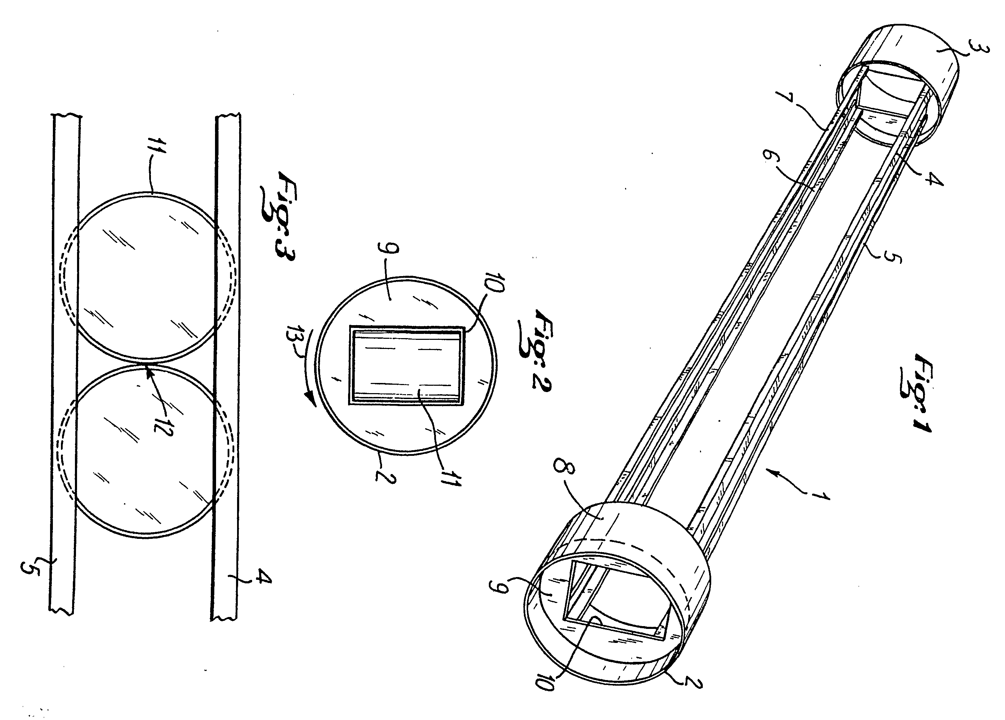

En se reportant au dessin, on voit que le panier 1 (figure 1) selon l'invention présente deux extrémités cylindriques 2 et 3 reliées entre elles par quatre cornières 4, 5, 6, 7 constituant une armature parallélépipédique. Chacune des extrémités du panier présente une surface externe cylindrique 8 et sensiblement en son milieu une cloison 9. Cette cloison 9 est munie d'une ouverture centrale 10 correspondant aux dimensions d'une boîte 11 (figure 2). Aux quatre angles de cette ouverture sont soudées les cornières 4 à 7.

Dans le panier selon l'invention, les boîtes sont chargées debout, c'est-à-dire avec leur axe longitudinal perpendiculaire à l'axe longitudinal du panier, et non plus couchées avec leur axe confondu avec l'axe du panier, comme dans les installations connues. De ce fait, elles ne sont plus en contact par leurs faces circulaires en regard, mais pratiquement par une génératrice. Comme de plus, les boîtes cylindriques présentent la plupart du temps un bord saillant dû au sertissage, c'est seulement par un point 12 (figure 3) de ce bord qu'elles sont en contact. Il en résulte que pratiquement toute la surface de la boîte entre en jeu pour assurer les échanges thermiques nécessaires à la stérilisation du produit alimentaire contenu dans la boîte.

En conséquence, la durée du processus de stérilisation peut être réduite pour une même qualité de la stérilisation.

Par ailleurs, lorsque le panier tourne sur lui-même autour de son axe longitudinal (flèche 13 de la figure 2), les boîtes sont entraînées en rotation bout pour bout et le produit contenu dans les boîtes est brassé, ce qui assure de meilleurs échanges thermiques et améliore la qualité de la stérilisation.

Là encore, on réalise une économie de temps de stérilisation, donc de consommation de vapeur dans l'enceinte, c'est-à-dire d'énergie de chauffage, et enfin de prix de revient.

Dans l'exemple de réalisation représenté et décrit, l'ouverture 10 est rectangulaire car c'est la forme qui correspond le plus fréquemment à celle des boîtes ; mais il va de soi qu'elle est nullement impérative. Ainsi pour des boîtes dont la hauteur est égale au diamètre, l'ouverture serait carrée.

Par ailleurs, on a représenté quatre cornières pour maintenir les boîtes dans le panier. Il va de soi qu'on peut matérialiser une armature parallélépipédique avec seulement deux cornières disposées à deux angles opposés de l'ouverture 10, sans sortir du cadre de l'invention.

## Revendications

1. Panier tubulaire pour la stérilisation des produits alimentaires en boîtes métalliques cylindriques, du type dans lequel les boîtes sont disposées jointives sur l'axe longitudinal du panier, caractérisé en ce qu'il comporte : deux extrémités (2, 3) cylindriques, et, entre elles une armature parallélépipédique constituée de cornières (4-7) longitudinales susceptibles de recevoir les boîtes cylindriques (11) disposées avec leur axe longitudinal perpendiculaire à l'axe longitudinal du panier (1), de façon à maintenir les boîtes dans une position relative fixe par rapport au panier.

2. Panier selon la revendication 1, caractérisé en ce que chaque extrémité cylindrique (2, 3) du panier (1) comporte une cloison (9) munie d'une ouverture (10) correspondant aux dimensions d'une boîte (11), et aux angles de laquelle sont fixées les cornières (4-7).

## Claims

1. A tubular basket for the sterilization of food products in cylindrical metallic cans, of the type in which the cans are disposed contiguously on the basket longitudinal axis, characterized in that it comprises : two cylindrical ends (2, 3), and, between them a parallelepipedal framework formed of longitudinal angle-pieces (4-7) adapted for receiving the cylindrical cans (11) disposed with their longitudinal axis perpendicular to the longitudinal axis of basket (1), so as to maintain the cans in a fixed relative position with respect to the basket.

2. A basket according to claim 1, wherein each cylindrical end (2, 3) of basket (1) includes a wall (9) formed with an opening (10) matching the dimensions of can (11), and at the corners of which are fixed the angle-pieces (4-7).

## Ansprüche

1. Röhrenförmiger Korb zur Sterilisierung von Lebensmittelt, die in zylinderförmigen Blechdosen verpackt sind, bei dem die Blechdosen nebeneinander längs der Längsachse des Korbes angeordnet sind, dadurch gekennzeichnet, daß er folgendes aufweist : zwei zylindrische Endstücke (2, 3), und zwischen diesen eine parallelepipedförmige, aus länglichen Winkelprofilen (4-7) bestehenden Einfassung, die geeignet ist, die zylinderförmigen Blechdosen (11) in der Lage aufzunehmen, in der ihre Längsachse senkrecht zur Längsachse des Korbes (1) liegt, derart, daß die Blechdosen in einer festen Position in Bezug auf den Korb gehalten werden.

2. Korb nach Anspruch 1, dadurch gekennzeichnet, daß jedes zylindrische Endstück (2, 3) des Korbes (1) mit einer Wand (9) versehen ist, die eine Öffnung (10) aufweist, die den Abmessungen einer Blechdose entspricht und an deren Ecken die Winkelprofile (4-7) befestigt sind.

*Fig. 1*

*Fig. 2*

*Fig. 3*